# EUROPEAN PATENT SPECIFICATION

(11) **EP 1 812 094 B1**
(45) Date of publication and mention of the grant of the patent: **17.08.2011**
(21) Application number: 05823244.8
(22) Date of filing: 16.11.2005
(51) Int. Cl.: A61M 1/10

(54) **Remote data monitor for heart pump system**
Datenmonitor auf Abstand für ein Herzpumpensystem
Appareil de surveillance à distance des données pour système de pompe cardiaque

(30) Priority: 16.11.2004 US 522874 P
(43) Date of publication of application: 01.08.2007
(73) Proprietor: Micromed Technology, Inc., Houston, TX 77054 (US)
(72) Inventor: MORELLO, Gino MICROMED CARDIOVASCULAR, Houston, TX 77054 (US)
(74) Representative: Taor, Simon Edward William
(86) International application number: PCT/US2005/041743
(87) International publication number: WO 2006/055745

(56) References cited:
- WO-A2-2005/091860
- US-A- 3 955 557
- US-A- 4 370 983
- US-A- 6 149 683
- US-A1- 2002 183 693
- US-A1- 2003 069 465
- US-A1- 2004 039 243

## Description

### CROSS REFERENCE TO RELATED APPLICATION

This application claims the benefit of U.S. Provisional Patent Application No. 60/522,874, entitled "REMOTE DATA MONITOR FOR HEART PUMP SYSTEM," filed on November 16, 2004, which is incorporated by reference.

### BACKGROUND

The invention relates generally to heart pump systems and, more specifically, to a remote monitor for such pumps.

Implantable blood pump systems are generally employed either to completely replace a human heart that is not functioning properly, or to boost blood circulation in patients whose heart still functions but is not pumping blood at an adequate rate. Known implantable blood pump systems are primarily used as a "bridge to transplant." In other words, existing blood pump system applications are mainly temporary fixes, intended to keep a patient alive until a donor is available. However, the shortage of human organ donors, coupled with improvements in blood pump reliability make long-term, or even permanent blood pump implementations a reality.

Despite this need, existing implantable pump systems have not been satisfactory for long term use. Known systems of the continuous flow type are designed primarily for use in a hospital setting. These systems typically include the implanted pump device, a power source such as a rechargeable battery, a motor controller for operating the pump motor, and an external operator console. While some existing implantable pump systems allow for operation while decoupled from the operator console, operating these systems "stand-alone" can be a risky endeavor. This is due, at least in part, to the lack of an adequate user interlace when the system is decoupled from the console.

Moreover, prior blood pump systems are not conducive to long-term use outside an institutional setting. Known systems often require a large, fixed operator console for the system to function. While prior art operator consoles may be cart mounted to be wheedled about the hospital, at home use of known systems is difficult at best Other problems of prior pump systems that have limited their mobility and use to relatively shott times are related to motor controller size and shape limitations.

US 6,149,683 discloses a power system for an implantable heart pump. The system includes two batteries, a microprocessor controller, two motor drivers, a multiplexer, two stators and a TET coil.

US 2003/0069465 discloses a controller modules for an implantable pump system which has a pump motor. The controller module includes a processor, a motor controller electrically coupled to the processor and adapted to power the pump motor such that the pump motor operates at a desired speed.

WO 2005/091860 discloses a method and apparatus for direct mechanical ventricular actuation with favourable conditioning and minimal heart stress.

Thus, there is a need for a pump control system that addresses the shortcomings associated with the prior art.

### SUMMARY

The invention is set out in the claims. In accordance with certain teachings of the present disclosure, a pump control system includes a controller module for controlling a pump, such as an implantable blood pump. A remote monitor is adapted to communicate with the controller module via a wireless communications medium, such as a low-power radio frequency link. The remote monitor provides a user interface similar or Identical to the controller module, providing a user the ability to remotely monitor the pump's performance and to respond to alarms.

### BRIEF DESCRIPTIOM OF THE DRAWINGS

Other objects and advantages of the invention will become apparent upon reading the following detailed description and upon reference to the drawings in which:

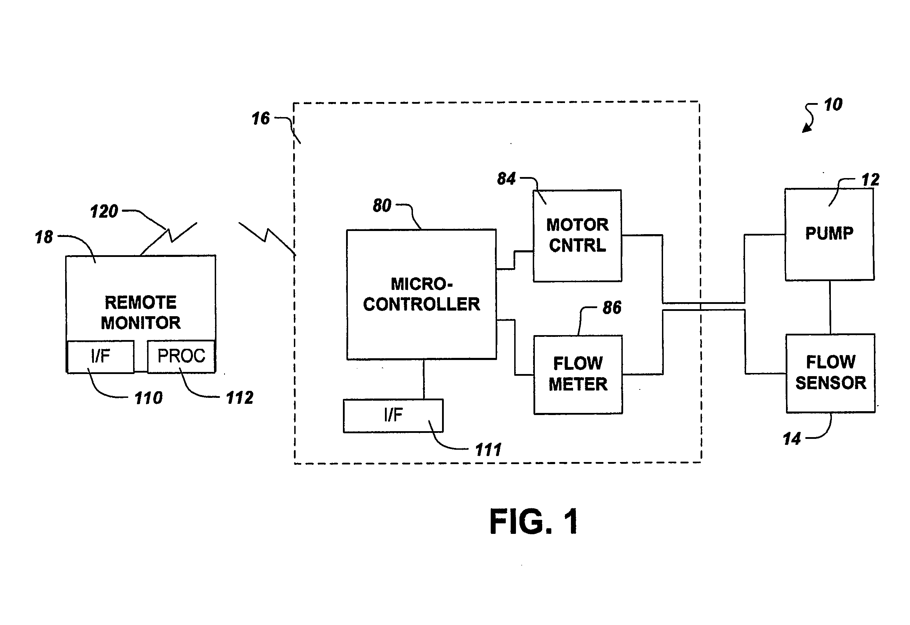

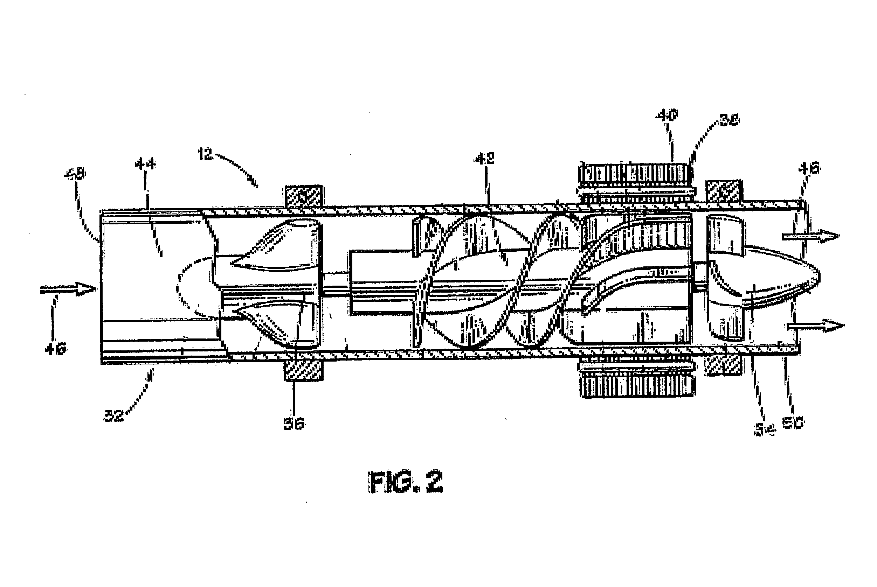

Figure 1 is a block diagram of a pump system in accordance with teachings of the present disclosure. Figure 2 illustrates an exemplary implantable heart pump in accordance with an embodiment of the system disclosed herein.

While the invention is susceptible to various modifications and alternative forms, specific embodiments thereof have been shown by way of example in the drawings and are herein described in detail. It should be understood, however, that the description herein of specific embodiments is not intended to limit the invention to the particular forms disclosed, but on the contrary, the intention is to cover all modifications, equivalents, and alternatives falling within the scope of the invention as defined by the appended claims.

### DETAILED DESCRIPTION

Illustrative embodiments of the invention are described below. In the interest of clarity, not all features of an actual implementation are described in this specification. It will of course be appreciated that in the development of any such actual embodiment, numerous implementation-specific decisions must be made to achieve the developers' specific goals, such as compliance with system-related and business-rotated constraints, which will vary from one implementation to another. Moreover, it will be appreciated that such a development effort night be complex and time-consuming, but would nevertheless be a routine undertaking for those of ordinary skill in the art having the benefit of this disclosure.

Turning to the figures, and in particular to Fig. 1, a ventricle assist device (VAD) system 10 in accordance with an embodiment of the present invention is illustrated. The VAD system 10 includes components designed to be implanted within a human body and components external to the body. The components of the system 10 that are implantable include a pump 12 and a flow sensor 14. The external components include a portable controller module 16 and a remote monitor module 18. The implanted components are connected to the controller module 16 via a percutaneous cable. The controller module 16 may be mounted to a support device, such as a user's belt 23 or to a vest worn by the user, for example. Additional components of a VAD system are shown and described in U.S. Patent No. 6,183,412, which is incorporated by reference.

The controller module 16 includes a processor, such as a microcontroller 80, which in one embodiment of the invention is a model PIC16C77 microcontroller manufactured by Microchip Technology. The microcontroller 80 includes a nmltiple channel analogue to digital (A/D) converter, which receives indications of motor parameters from the motor controller 84. Thus, the controller module 16 may monitor parameters such as instantaneous motor current, the DC component or mean value of the motor current, and motor speed.

The embodiment shown in FIG. 1 further includes an integral flow meter 86. At least one flow sensor 14 is implanted down stream of the pump 12. Alternately, a flow sensor 14 may be integrated with the pump 12. The flow meter 86 is coupled between the implanted flow sensor 14 and the microcontroller 80. The flow meter 86 receives data from the flow sensor 14 and outputs flow rate date to the microcontroller 80, allowing the system to monitor instantaneous flow rate.

The VAD System 10 may incorporate an implantable continuous-flow blood pump, such as the various embodiments of axial flow pumps disclosed in U.S. Patent No. 5,527,159 or in U.S. Patent No. 5,947,892, both of which are incorporated herein by reference in their entirety. An example of a blood pump suitable for use in an embodiment of the invention is illustrated in FIG. 2. The exemplary pump 12 includes a pump housing 32, a diffuser 34, a flow straightener 36, and a brushless DC motor 38, which includes a stator 40 and a rotor 42. The housing 32 includes a flow tube 44 having a blood flow path 46 therethrough, a blood inlet 48, and a blood outlet 50.

The stator 40 is attached to the pump housing 32, is preferably located outside the flow tube 44, and has a stator field winding 52 for producing a stator magnetic field. In one embodiment, the stator 40 includes three stator windings and may be three phase "Y" or "Delta" wound. The rotor 42 is located within the flow tube 44 for rotation in response to the stator magnetic field, and includes an inducer 58 and an impeller 60. Excitation current is applied to the stator windings 52 to generate a rotating magnetic field. A plurality of magnets 62 are coupled to the rotor 42. The magnets 62, and thus the rotor 42, follow the rotating magnetic field to produce rotary motion.

The remote data monitor (RDM) 18 is a small portable handheld device that includes a processing device 112 and a user interface 110 that effectively replicates the user interface 111 of the controller module 16 remotely via a wireless communication link 120. In an exemplary embodiment, the wireless link 120 is a low-power radio frequency (RF) link usable over a maximum distance of approximately 300 feet (100 meters) using the system's standard antenna configuration. While in use, the device provides the user with the ability to remotely monitor the VAD pump's 12 performance and to respond to alarms. The pump controller 16 and remote monitor(s) 18 may be programmed via the clinical data acquisition system or the remote monitor 18 may be programmed telemetrically by the pump controller 16, for example.

The wireless link 120 includes antennas, which my suitably comprise simple monopoles. The system's antennas maybe constructed from ferrite rods or with traces on the system's internal printed circuit board. Integral antennas may be used exclusively, or external antennas may be employed for increased range capability, or a combination of integral and external antennas may be used.

The integrity of the link 120 will be continuously verified while the device is in operation. In the event that the remote monitor 18 is located too far from the VAD controller 16, the RF link becomes "noisy" or unusable, or if the monitor's 18 batteries are low, the VAD controller 16 will continue to function and alarm normally. More specifically, in exemplary implementations, the remote monitor 18 periodically transmits status information back to the pump controller 16 to confirm proper link operation. The remote monitor 18 may monitor and display received signal strength information, and the pump controller 16 can increase or decrease its transmitter output power proportional to the signal strength reported back from the remote monitor 18.

The carrier may be angle modulated (i.e. frequency modulated (FM) or phase modulated (PM)) to minimize the effects of external noise induced errors. Alternatively, the carrier may be amplitude modulated (AM) to maximize battery life. Forward error correction techniques may be used to maximize the integrity of the communication link. Spread spectrum techniques are used to further minimize externally induced noise from compromising the communication link between the pump controller and corresponding remote monitor. The receiver may request that a transmission be retransmitted in the event of an error. In typical installations, transmissions are within the US and European ISM (i.e. Industrial, Scientific, Medical) band.

Multiple controller 16 and remote monitor 18 pairs may be used in close proximity to one another. Each pump controller 16 and corresponding remote monitor 18 may communicate on a designated frequency or frequency pair or on the same frequency or frequency pair. Transmitted data packets contain the address of the intended remote monitor 16. Each pump controller 16 first "listens" to confirm if another pump controller 16 is transmitting. In the event there is no other transmission, the pump controller 16 may begin transmitting and, conversely, in the event another transmission is "heard" the pump controller 16 will wait for the channel to be clear. In other implementations, one pump controller 16 may broadcast to several remote monitors 16 (e.g. one with the patient, one with the caregiver).

The data transmitted between the pump controller 16 and remote monitor 18 may be encoded such that the remote monitor 18 only responds to data transmitted with a unique address or to transmissions containing the correct address. A hardware or software based correlator may be used to identify the address.

In certain embodiments, the remote monitor 18 is approximately 3 inches wide by 2 inches high by 1 inch thick, weighs less than 5 oz., includes a wrist-strap such that it may be worn by the caregiver or patient on the wrist, and includes a combination belt clip/tilt stand for use on the patient's or caregiver's belt or nightstand. The device 18 may be powered from an internal rechargeable battery to be completely portable or it may be plugged into the ac mains using an optional power adapter. Additionally, the device 18 may be plugged into an automotive power outlet for continuous operation while on long trips in an automobile or airplane. The device 18 will support simultaneous charging of the internal battery while monitoring the VAD controller 16 (e.g. at night while patient and parent/caregiver are sleeping).

The remote monitor's 18 user interface is identical to the VAD controller 18 interface and includes a tricolor light emitting diode (LED) backlit graphic liquid crystal display (LCD) to display multi-lingual diagnostic and emergency messages, a sealed two-button keypad with tactile feedback and rim-embossing to silence alarms and scroll through diagnostic message displays, three bicolor LEDs indicating individual battery status and fail-safe mode operation, two distinct, variable pitch, variable loudness audible enunciators, and an optional audible voice output for diagnostic and emergency alarms.

The backlit LCD can indicate functional pump information to the patient and/or caregiver, and in exemplary embodiments, the backlight utilizes multiple colors to convey functional and alarm information to the patient and caregiver (e.g. green=normal, yellow=diagnostic alarm, red=emergency alarm).

The audible alarms may be elicited through piezo buzzer enunciators. The variable loudness audible enunciators maybe be operated such that the pitch and/or volume changes proportionally to the length of time that the alarm is activated. The audible voice output may be elicited through a voice coil type speaker element. A natural language speech synthesizer may be employed, including a phoneme based speech synthesizer enabling audible speech to be generated in a multiplicity of languages. Further, the natural language voice's pitch and cadence may be programmed to simulate a male or female adult voice based on the patient or caregiver's preference. Still further, the natural language voice output's pitch and cadence may be programmed to simulate a less-intimidating child's voice for pediatric cases. A motor with integral eccentric may be enabled to vibrate during any alarming condition to help in alerting the patient or caregiver.

Optionally, in pediatric applications, a wireless audio channel may be added to integrate the functionality of a commercial "baby monitor" into the system. A transmitter with a microphone or other sound detecting device transmits audio signals to a receiver integrated into the remote monitor 18, which further includes an output device such as a speaker. This minimizes the number of different systems the parent or caregiver must use and manage. This function would also include a volume control to allow the patent or caregiver to set the device's output to the desired audio level.

According to an example, there is provided a method of operating a blood pump, comprising: connecting a controller module to a blood pump; and transmitting data between a monitor module and the controller module via a wireless communications medium.

Optionally according to said method transmitting data includes transmitting functional and alarm messages. Optionally the method further comprises powering the pump such that the pump operates at a desired speed; outputting digital representations of pump operating parameters on a user interface of the controller module; transmitting the pump operating parameters to the monitor module; and displaying the pump operating parameters on a user interface of the monitor module.

Optionally according to said method, transmitting data includes transmitting via a bi- directional radio frequency (RF) link.

Optionally the method further comprises attaching the monitor module to a user's wrist.

Optionally the method further comprises attaching the monitor module to a user's belt.

Optionally the method further comprises transmitting status information from the module to the controller module.

Optionally according to said method, transmitting status information includes transmitting received signal strength information, and optionally the method further comprises varying the transmit output power of the controller module in response to the received signal strength information.

Optionally according to said method a plurality of controller modules are connected to a plurality of corresponding blood pumps, and one monitor module communicates with the plurality of controller modules.

Optionally according to said method, a plurality of controller modules are connected to a plurality of corresponding blood pumps, and each of the controller modules communicates with a corresponding monitor module on a predetermined frequency, and optionally data transmitted from the controller module includes the address of the intended monitor module.

Optionally according to said method, a plurality of monitor modules communicate with a single controller module.

Optionally according to said method the controller module verifies that no other controller module is transmitting before transmitting data to the monitor module.

The above description of exemplary embodiments are made by way of example and not for purposes of limitation. Many variations may be made to the embodiments and methods disclosed herein without departing from the scope of the present invention. The present invention is intended to be limited only by the scope of the following claims.

## Claims

1. A pump control system comprising:
a controller module (16) connectable to an implanted heart pump (12); and
a monitor module (18) adapted to communicate with the controller module (16) via a wireless communications medium, **characterised in that** both the controller module (16) and the monitor module (18) are external to a body in which the pump (12) resides.

2. The pump control system of claim 1, wherein the controller module includes:
a processor (80);
a motor controller (84) electrically coupled to the processor (80), the motor controller (84) adapted to power the pump motor (38) such that the pump motor (38) operates at a desired speed, the motor controller (84) adapted to output digital representations of pump motor operating parameters to the processor (80);
a user interface (111) coupled to the processor (80); and
wherein the monitor module (18) includes a user interface (110).

3. The pump control system of claim 2, wherein the controller module user interface (111) matches the monitor module user interface (110).

4. The pump control system of claim 1, wherein the wireless communications medium is a bi-directional radio frequency (RF) link (120).

5. The pump control system of claim 1, wherein monitor module (18) includes a device for attaching to a user's wrist.

6. The pump control system of claim 1, wherein monitor module (18) includes a device for attaching to a user's belt.

7. The pump control system of claim 1, wherein monitor module (18) includes a device for receiving and outputting audio signals.

8. The pump control system of claim 1, wherein monitor module (18) is programmed to periodically transmit status information to the controller module (16).

## Patentansprüche

1. Pumpensteuersystem, das aufweist:
einen Reglermodul (16), der mit einer implantierten Herzpumpe (12) verbunden werden kann; und
einen Monitormodul (18), der ausgebildet ist, um mit dem Reglermodul (16) mittels eines drahtlosen Kommunikationsmediums eine Verbindung herzustellen, **dadurch gekennzeichnet, dass** sich sowohl der Reglermodul (16) als auch der Monitormodul (18) außerhalb eines Körpers befinden, in dem die Pumpe (12) untergebracht ist.

2. Pumpensteuersystem nach Anspruch 1, bei dem der Reglermodul umfasst:
einen Prozessor (80);
einen Motorregler (84), der elektrisch mit dem Prozessor (80) verbunden ist, wobei der Motorregler (84) ausgebildet ist, um den Pumpenmotor (38) mit Energie zu versorgen, so dass der Pumpenmotor (38) mit einer gewünschten Drehzahl arbeitet, wobei der Motorregler (84) ausgebildet ist, um digitale Darstellungen der Betriebsparameter des Pumpenmotors an den Prozessor (80) auszugeben;
eine Benutzerschnittstelle (111), die mit dem Prozessor (80) verbunden ist; und
wobei der Monitormodul (18) eine Benutzerschnittstelle (110) umfasst.

3. Pumpensteuersystem nach Anspruch 2, bei dem sich die Benutzezschnittstelle (111) des Reglermoduls an die Benutzerschnittstelle (110) des Monitormoduls anpasst.

4. Pumpensteuersystem nach Anspruch 1, bei dem das drahtlose Kommunikationsmedium eine in zwei Richtungen arbeitende Hochfrequenz(HF)-Verbindung (120) ist.

5. Pumpensteuersystem nach Anspruch 1, bei dem der Monitormodul (18) eine Vorrichtung für ein Befestigen am Handgelenk eines Benutzers umfasst.

6. Pumpensteuersystem nach Anspruch 1, bei dem der Monitormodul (18) eine Vorrichtung für ein Befestigen am Gürtel eines Benutzers umfasst.

7. Pumpensteuersystem nach Anspruch 1, bei dem der Monitormodul (18) eine Vorrichtung für das Empfanden und Ausgeben von Audiosignalen umfasst.

8. Pumpensteuersystem nach Anspruch 1, bei dem der Monitormodul (18) programmiert ist, um eine Zustandsinformation zum Reglermodul (16) periodisch zu übertragen.

## Revendications

1. Système de commande de pompe, comprenant :
un module de commande (16) pouvant être connecté à une pompe cardiaque implantée (12) ; et
un module de surveillance (18), adapté pour communiquer avec le module de commande (16) par l'intermédiaire d'un moyen de communication sans fil, **caractérisé en ce que** le module de commande (16) et le module de surveillance (18) sont agencés à l'extérieur d'un corps dans lequel réside la pompe (12).

2. Système de commande de pompe selon la revendication 1, dans lequel le module de commande englobe :
un processeur (80);
un moyen de commande du moteur (84), accouplé électriquement au processeur (80), le moyen de commande du moteur (84) étant adapté pour actionner le moteur de la pompe (38), de sorte que le moteur de la pompe (38) fonctionne à une vitesse voulue, le moyen de commande du moteur (84) étant adapté pour transmettre des représentations numériques des paramètres opérationnels du moteur de la pompe vers le processeur (80) ;
une interface utilisateur (111), accouplée au processeur (80) ; et
le module de surveillance (18) englobant une interface utilisateur (110).

3. Système de commande de pompe selon la revendication 2, dans lequel l'interface utilisateur du module de commande (111) est adaptée à l'interface utilisateur (110) du moyen de surveillance.

4. Système de commande de pompe selon la revendication 1, dans lequel le moyen de communication sans fil est une liaison radiofréquence (RF) bidirectionnelle (120).

5. Système de commande de pompe selon la revendication 1, dans lequel le module de surveillance (18) englobe un dispositif permettant la fixation sur le poignet d'un utilisateur.

6. Système de commande de pompe selon la revendication I, dans lequel le module de surveillance (18) englobe un dispositif permettant la fixation à une ceinture d'un utilisateur.

7. Système de commande de pompe selon la revendication 1, dans lequel le module de surveillance (18) englobe un dispositif pour recevoir et transmettre des signaux audio.

8. Système de commande de pompe selon la revendication 1, dans lequel le module de surveillance (18) est programmé pour transmettre périodiquement des informatisons d'état au module de commande (16).
